# EUROPEAN PATENT SPECIFICATION

(11) **EP 1 191 943 B1**
(45) Date of publication and mention of the grant of the patent: **11.11.2009**
(21) Application number: 00937154.3
(22) Date of filing: 14.06.2000
(51) Int. Cl.: A61K 39/385, A61K 47/48, C07K 14/47

(54) **Anti-allergic peptides**
Anti-allergene Peptide
Peptides antiallergiques

(30) Priority: 17.06.1999 IL 13052699
(43) Date of publication of application: 03.04.2002
(60) Divisional application: 07022898.6
(73) Proprietor: Ramot at Tel-Aviv University Ltd., Tel-Aviv 69975 (IL)
(72) Inventor: EISENBERG, Ronit, 74047 Ness-Ziona (IL); RAZ, Tamar, 48056 Rosh Haayin (IL)
(74) Representative: Wachenfeld, Joachim
(86) International application number: PCT/IL2000/000346
(87) International publication number: WO 2000/078346

(56) References cited:
- US-A- 5 807 746
- SINGH R ET AL: "Antiallergic/antiasthmatic activity of oligopeptide related to IgE." PHARMACOLOGICAL RESEARCH : THE OFFICIAL JOURNAL OF THE ITALIAN PHARMACOLOGICAL SOCIETY. MAY 1998, vol. 37, no. 5, May 1998 (1998-05), pages 353-356, XP002292857 ISSN: 1043-6618
- ARIDOR ET AL.: 'Activation of exocytosis by the heterotrimeric G protein G13' SCIENCE vol. 262, no. 5139, 03 December 1993, pages 1569 - 1573, XP000953320

## Description

### FIELD OF THE INVENTION

The present invention discloses novel anti-allergic agents, and in particular, peptidic molecules, including at least a first segment which is competent for cell penetration and a second segment which is able to reduce or abolish mast cell degranulation, and in particular to reduce or abolish allergy mediators such ass histamine secretion from mast cells as characterized in the claims.

### BACKGROUND OF THE INVENTION

Allergic diseases, including nasal allergy, asthma, urticaria and angioedema, are among the most common diseases encountered by physicians in their clinical practice. Allergy refers to certain diseases in which a wide spectrum of biologically active substances, released from activated mast cells, cause tissue inflammation and organ dysfunction. In essence, any allergic reaction may lead to tissue damage in one or more target organs (see for example Lichtenstein 1993; full references are given in the list at the end of the specification).

On the cellular level, mast cells are significant contributors to the allergic reaction and are packed with 500 to 1000 granules in which the mediators of the inflammatory reactions are stored. These include vasoactive mediators such as histamine, chemotactic mediators and proteolytic enzymes. In addition, following the activation of mast cells, a number of mediators are generated de novo and released. These include arachidonic acid metabolites such as leukotrienes and prostaglandins and a number of multifunctional cytokines. Mast cell derived factors also recruit and activate additional inflammatory cells, such as eosinophils, neutrophils and mononuclear cells. Therefore, mast cell derived mediators possess all the requisite properties to induce the symptoms of itching, swelling, coughing and choking that are associated with an allergic reaction (Bienenstock et al., 1987). These mediators are released in response to processes which occur through a number of different pathways within mast cells. Thus, therapeutic treatments for allergy and related inflammatory conditions must intervene at some point in the allergic pathway in order to be effective.

Current therapies against allergy include H₁ and H₂ blockers, which block the biological activities of histamine, and leukotriene antagonists, which block the biological activities of leukotrienes. Examples include chlorpheniramine, azatidine, ketotifen, loratidine, montelukast sodium and others. However, anti-histamines or anti-leukotrienes can not counteract the inflammatory reactions effected by the additional mediators released alongside histamine and leukotrienes . Therefore, these drugs cannot provide a reliable protection against allergy.

A better allergy treatment would block the secretory process by preventing mast cell degranulation. Drugs which are currently available for this purpose include hydrocortisone and disodium cromoglycate. However, disodium cromoglycate cannot inhibit all types of histamine secretion, and is not always completely effective. Steroids, on the other hand, are effective for blocking mast cell degranulation, but have many unacceptable side effects. Therefore, therapeutic agents which could prevent mast cell degranulation without significant side effects, and could thus prevent or significantly reduce the occurrence of clinical symptoms associated with allergy, such as neurogenic inflammation (see below for details), would be very useful for the treatment of allergy and related conditions.

Mast cell degranulation is a complex process involving at least two different pathways. Mast cells secrete their granular contents in a process of regulated exocytosis (degranulation) by two major pathways, the IgE (immunoglobulin E) dependent pathway and the IgE independent pathway. The IgE dependent pathway is invoked in response to an immunological trigger, brought about by aggregation of the high affinity receptors (F_{c}εRJ) for IgE, which are present on the cell surface of mast cells. This response involves crosslinking of cell bound IgE antibodies by the corresponding antigens (allergens).

The IgE-independent or peptidergic pathway is invoked in response to a number of polycationic compounds, collectively known as the basic secretagogues of mast cells. These compounds include the synthetic compound 48/80, naturally occurring polyamines and positively charged peptides, such as the neurotransmitter substance P (Ennis et al., 1980; Sagi-Eisenberg 1993; Chahdi et al., 1998).

The ability of substance P to induce mast cell degranulation, together with the observed presence of mast cells clustered around nerve endings which contain substance P, implicate mast cells as the mediators of substance-P induced neurogenic inflammation (Foreman 1987a,b; Pearce et al., 1989). It is well established that in the skin and elsewhere neurogenic inflammation, through the release of neurotransmitters such as substance P, is a contributor to a variety of diseases such as acute urticaria, psychogenic asthma, interstitial cystitis, bowel diseases, migraines, multiple sclerosis and more (Reviewed by Theoharides 1996). In addition, this IgE independent pathway of degranulation can also be evoked by snake, bee and wasp venoms, bacterial toxins and certain drugs such as opiates.

Although the signal transduction pathways by which mast cell degranulation is activated are not yet fully resolved, a number of cellular events have been shown to occur after stimulation of the mast cells. These include activation of phospholipases such as PLC, PLD and PLA₂, elevation of cytosolic Ca ²⁺ and activation of serine and tyrosine kinases (reviewed by Sagi-Eisenberg 1993). Within these processes, however, the involvement of GTP-binding proteins (G-proteins) is well established. For example, the introduction of nonhydrolyzable analogues of GTP, such as GTP-γ-S, into ATP⁻⁴ permeabilized mast cells, stimulates PLC activity and degranulation.

From these observations and other observations, the involvement of at least two different G-proteins, one involved in PLC and Ca²⁺ activation (Gp) and one directly regulating exocytosis (G_{E}), has been suggested (Gomperts et al., 1991; reviewed by Sagi-Eisenberg 1993). Indeed, it was subsequently demonstrated that basic secretagogues induce histamine secretion by interacting directly with G_{E}, a pertussis toxin-sensitive heterotrimeric G protein, in a receptor-independent manner (Aridor et al., 1990; Aridor & Sagi-Eisenberg 1990). This G-protein was subsequently identified as Gi₃, which appears to mediate the peptidergic pathway leading to exocytosis in mast cells. In particular, a synthetic peptide which corresponds to the C terminal sequence of Gαi₃ (KNNLKECGLY) was able to inhibit histamine release when introduced, into permeabilized mast cells (Aridor et al., 1993).

However, the cell membrane is generally impermeable to most peptides. Therefore, the use of a peptide as a therapeutic agent, directed against an intracellular target, requires a special mechanism to enable the peptide to overcome the membrane permeability barrier.

One approach is based on the fusion of the selected peptide with a specific hydrophobic sequence, comprising the "h" region of a signal peptide sequence. Examples of such hydrophobic regions are the signal sequence of the Kaposi fibroblast growth factor (AAVALLPAVLLALLAP; Lin et al., 1995) and the signal sequence within human integrin β₃ (VTVLALGALAGVGVG; reviewed by Hawiger 1997). Another approach is based on fusing the active anti-allergic peptide with a specific signal peptide sequence endowed with the membrane translocation properties of the homeodomain of Antennapedia, a *Drosophila* transcription factor (RQPKIWFPNRRKPWKK; Prochiantz 1996).

Specific importation of biologically active molecules into cells by linking an importation-competent signal peptide to the molecule of interest was disclosed in U.S. Patent No. 5,807,746, although only *in vitro* studies were described, such that the signal peptide was not shown to function *in vivo.* The signal peptide causes the entire complex to be imported into the cell, where theoretically the biologically active molecule could then have its effect. Although such direct importation could serve to target the therapeutic compound, and could therefore prevent or substantially reduce side effects through non-targeted system activity, the efficacy of the complex within the cell may be limited, such that the biologically active molecule may have little or no effect once imported into the cell. The variables which may affect the efficacy of the biologically active molecule include the effect of linking the molecule to the signal peptide, which may result in an inactive complex; unpredictable effects of the entire complex within the cell; and even the inability of the entire complex to be imported into the cell, despite the presence of the signal peptide.

In addition, identifying a suitable biologically active molecule for treatment of allergy may also be difficult. For example, linking a non-peptide molecule, such as a known secretion-blocking compound, to a signal peptide is both difficult and may result in an unstable molecule. A peptide could be used as the secretion-blocking compound, but then such a peptide must be carefully selected and tested. Finally, the entire complex would require testing, particularly *in vivo,* since the ability to penetrate a cell in tissue culture does not necessarily predict the efficacy of the complex in a human or animal subject. U.S. Patent No. 5,807,746 therefore suffers from the drawback that only *in vitro* data is disclosed, such that the effect of the signaling peptides *in vivo,* alone or as part of a complex, is not known. Thus, suitable, targeted, specific therapeutic agents for the treatment of allergy are not currently available and are potentially complex and difficult to develop.

There is therefore a need for, and it would be useful to have, a therapeutic agent for the treatment of allergy and related inflammatory conditions, which would block mast cell degranulation and hence the release of histamine, but which would be specifically targeted to the degranulation pathway and which would therefore have few side effects;

### SUMMARY OF THE INVENTION

According to the present invention, there is provided a therapeutic agent for treating an allergic condition comprising anti-allergic complex, peptide having an amino acid sequence AAVALLPAVLLALLAP molecule having at least a first competent for importation of the molecule into mast cells in vivo, and a second peptide having an amino acid sequence KNNLKECGLY or KENLNDCGLF for having an anti-allergic effect within the mast cells, the first peptide being joined to the second segment via a peptide bond, whereby the complex molecule is capable of exerting its anti-allergic effect in vivo.

Preferably, the second peptide provides the anti-allergic effect by at least significantly reducing degranulation of the mast cells. peptide has an amino acid sequence AAVALLPAVLLALLAPKNNLKACGLY.

According to another particularly preferred embodiment of the present invention, the molecule is a peptide having an amino acid sequence AAVALLPAVLLALLAPKENLKDCGLF.

Preferably, the allergic condition is selected from the group consisting of nasal allergy, an allergic reaction in an eye of the subject, an allergic reaction in the skin of the subject, acute urticaria, psoriasis, psychogenic or allergic asthma, interstitial cystitis, bowel diseases, migraines, and multiple sclerosis. More preferably, the therapeutic agent is to be administered by topical administration. Most preferably, the topical administration is to the skin of the subject.

According to another preferred embodiment of the present invention, the therapeutic agent is to be administered by inhalation or intranasally. Alternatively and preferably, the therapeutic agent is to be administered orally or systemically via parenteral routes, including in depot form, or by any other suitable route of administration.

According to a currently more preferred embodiment of the present invention, the molecule comprises a peptide having an amino acid sequence selected from the group consisting of :
i) AAVALLPAVLLAL.LAPKNNLICECGLY;
ii) AAVALLPAVLLALLAPKENLKDCGLF;
ill) Succinyl AAVALLPAVLLALLAPKNNLKECGLY
iv) Cyclic- AAVALLPAVLLALLAPKNNLKECGLY; and mixtures thereof.

More preferably the therapeutic agent further comprises a second molecule, the second molecule comprising a peptide having an amino acid sequence

Also described herein is a method for promoting importation of a molecule into a cell of a subject *in vivo*, the method comprising the steps of:
(a) attaching a leader sequence to the molecule, the leader sequence being a peptide having an amino acid sequence AAVALLPAVLLALLAP, to form a complex; (b) administering the complex to the subject; and (c) importing the complex into the cell through the leader sequence, such that the molecule is imported into the cell.

Hereinafter, the term "biologically active" refers to molecules, or complexes thereof, which are capable of exerting an effect in a biological system. Hereinafter, the term "fragment" refers to a portion of a molecule or a complex thereof, in which the portion includes substantially less than the entirety of the molecule or the complex thereof.

Hereinafter, the term "amino acid" refers to both natural and synthetic molecules which are capable of forming a peptide bond with another such molecule. Hereinafter, the term "natural amino acid" refers to all naturally occurring amino acids, including both regular and non-regular natural amino acids. Hereinafter, the term "regular natural amino acid" refers to those alpha amino acids which are normally used as components of a protein. Hereinafter, the term "non-regular natural amino acid" refers to naturally occurring amino acids, produced by mammalian or non-mammalian eukaryotes, or by prokaryotes, which are not usually used as a component of a protein by eukaryotes or prokaryotes. Hereinafter, the term "synthetic amino acid" refers to all molecules which are artificially produced and which do not occur naturally in eukaryotes or prokaryotes, but which fulfill the required characteristics of an amino acid as defined above. Hereinafter, the term "peptide" includes a chain of a sequence of amino acids, whether natural, synthetic or recombinant, wherein said amino acids are linked by peptide bonds. Hereinafter, the term "peptidomimetic" includes both peptide analogues and mimetics having substantially similar or identical functionality thereof, including analogues having synthetic and natural amino acids, wherein the peptide bonds may be replaced by other covalent linkages.

### BRIEF DESCRIPTION OF THE DRAWINGS

The invention is herein described, with reference to the accompanying drawings, wherein:
Figure 1 is a graph of the effect of different peptides on histamine secretion.
Figure 2 is a graph of the dose effect of compound 48/80 induced histamine release from intact mast cells.
Figure 3 is a graph of the inhibitory effect of peptides 1 any 4 on histamine secretion.
Figure 4 is a graph of the inhibitory effect of peptides 2 and 5 on histamine secretion.
Figure 5: Inhibitory effect of peptide 2 on histamine secretion.
Figure 6: Inhibitory effect of peptide 2 on substance P induced histamine secretion.
Figure 7: Blocking effect of peptide 5 on histamine secretion.
Figure 8: Effect of different peptides on histamine secretion.
Figure 9: Effect of peptide 20 (A) and peptide 21 (B) on histamine secretion, induced by compound 48/80.
Figure 10: Effect of succinylated peptide 2 (2-Suc) on histamine secretion.
Figure 11: A computerized model demonstrating 3D structure of the C-terminus sequence of Peptide 2 - KNNLKECGLY (A) and peptide 5m - KNNLKDCGLF (B).
Figure 12: Effect of peptide 2-Cyc on histamine secretion.
Figure 13: Effect of peptide 2 and peptide 2-Suc on IgE Induced histamine secretion.
Fig 14: Rat Skin Test demonstrates the wheals that developed as a result of intradermal injection of Vehicle (B,D,F) or compound 48/80 (A,C,E), after intradermal injection of vehicle (A,B) or two different concentrations of Peptide 2 (C,D and E,F).

### DESCRIPTION OF THE PREFERRED EMBODIMENTS

The present invention discloses a therapeutic complex for the specific, direct and targeted treatment of allergies and related inflammatory conditions, which comprises molecules having at least a first peptide which is competent for the importation of the complex into mast cells, and a second peptide which is able to block or significantly as characterized in the claims reduce mast cell degranulation and hence the release of histamine. The first segment is connected to the second segment via a peptide bood.

The first is a signal peptide. A signal peptide is a peptide which is capable of penetrating through the cell membrane, to permit the exportation and/or importation of proteins or peptides. Signal peptides described herein are those which are competent for the importation of proteins, peptides or other molecules into the cell. Such signal peptides generally feature approximately 10-50 amino acids, of which a majority are typically hydrophobic, such that these peptides have a hydrophobic, lipid-soluble portion. Signal peptides can also be selected according to the type of cell into which the complex is to be imported, such that signal peptides produced by a particular cell type, or which are derived from peptides and/or proteins produced by that cell type, can be used to import the complex into cells of that type. Examples of such signal peptides are described above and are also disclosed in U.S. Patent No. 5,807,746.

The second peptide has an anti-allergic effect, preferably by preventing mast cell degranulation, and hence the release of histamine from these mast cells. The peptide is derived from the C terminal sequence of Gaij, which appears to mediate the peptidergic pathway leading to exocytosis in mast cells. Also described herein are the second segment selected from a polypeptide.

The linker which connects the first peptide to the second peptide is a peptide bond. Futher described herein is that the linker which connects the first segment to the second segment.

The present invention also discloses therapeutic agents for treating allergies. Hereinafter, the term "treatment" includes both the prevention of the allergic condition, as well as the substantial reduction or elimination of allergic symptoms. Allergic conditions for which the therapeutic agents of the present invention are useful include, nasal allergy, irritation or allergic reactions in the eyes, allergic reactions in the skin including any type of allergen-induced rash or other skin irritation or inflammation, acute urticaria, psoriasis, psychogenic or allergic asthma, interstitial cystitis, bowel diseases, migraines, and multiple sclerosis.

Such treatment may be performed topically, for example for skin allergies and allergic reactions contact dermatitis in reaction to skin contact with an allergen; reactions to insect bites and stings; and skin reactions to systemic allergens, such as hives appearing after a food substance has been ingested by the subject. Alternatively and/or additionally, such treatment may be performed by systemic administration of the therapeutic complex. A preferred route of administration is oral, but alternative routes of administration include, intranasal, intraocular, sub-cutaneous and parenteral administration. Other routes of administration, and suitable pharmaceutical formulations thereof, are described in greater detail below.

As noted previously, the first and the second peptides are joined with a peptide bond.

According to the principles of the present invention, novel peptides, designated as peptides 1-6, were designed and synthesized to include distinct importation competent signal peptides as a first segment at the N-terminus (underlined) and the C-terminal sequences of Gαi₃ or Gαt at the C-terminus as a second segment. In the following list the non-underlined part is Gαi₃ for peptides 1-3, and Gαt for peptides 4-6:
1. VTVLALGALAGVGVGKNNLKECGLY
2. AAVALLPAVLLALLAPKNNLKECGLY
3. ROPKIWFPNRRKPWKKKNNLKECGLY
4. VTVLALGALAGVGVGKENLKDCGLF
5. AAVALLPAVLLALLAPKE NLKDCGLF
6. ROPKJWFPNRRKPWKKKENLKDCGLF

These peptides were then examined *in-vitro* for their ability to block compound 48/80 induced histamine secretion from purified rat peritoneal mast cells (Aridor *et al*., 1993). Peptides 2 and 5 were demonstrated as blockers of compound 48/80 induced histamine secretion from mast cells in a dose response effect. Peptide 2 was more potent than peptide 5, however when applied together both peptides showed, a synergistic effect for inhibiting histamine release. Peptides 1,3,4, and 6 showed no inhibition.

Peptides 2 and 5, and their homologues or structurally similar related compounds, could therefore be useful for non-immunological (IgE-independent) allergies, in which mast cell degranulation is mediated through the IgE-independent pathway from which the second segment of the above peptides was taken. Examples of such allergies include to neurogenic inflammation in the skin and elsewhere, including acute urticaria, psoriases, psychogenic asthma, interstitial cystitis, bowel diseases, migraines, and multiple sclerosis. In addition, the leader sequence (first segment) of peptides 2 and 5 could also be useful for the importation of a molecule into the cell of a subject *in vivo,* as such an effect had not been previously demonstrated in the background art.

### EXAMPLE 1

### TESTING OF PEPTIDES 1-6 IN VITRO

Peptides 1-6 of the present invention, as described above, were tested *in vitro* for their ability to block histamine secretion from mast cells. Rat peritoneal mast cells were chosen as the experimental model, since it was previously shown that both rat peritoneal and human skin mast cells release histamine in response to substance P by an IgE-independent mechanism (Devillier et al., 1986; Foreman 1987a,b; Columbo et al., 1996). It was also demonstrated that the same peptidergic pathway is involved in both rat peritoneal and human cutaneous mast cells (Mousli et al., 1994; Emadi-Khiav et al., 1995).

Compound 48/80 was chosen as the allergen since it is one of the polycationic compounds, collectively known as the basic secretagogues of mast cells. Compound 48/80 has been shown to induce degranulation of human mast cells. In particular, it is very active on skin mast cells. Compound 48/80 has been used as a diagnostic agent *in vivo* to assess the release ability of human mast cells, to determine the effectiveness of drugs against chronic urticaria and to study itch and flare responses in atopic dermatitis (Kivity et al., 1988; Goldberg et al., 1991). Therefore inhibition of compound 48/80 induced histamine release is applicable and relevant to prevention of allergy induced by other basic secretagogues such as substance P, snake, bee and wasp venoms, bacterial toxins and certain drugs such as opiates.

The ability of each of peptides 1-6 to inhibit mast cell degranulation, when induced by compound 48/80, was then tested. The experimental method was as follows.

### MATERIALS AND METHODS

### Peptide synthesis

Peptides were synthesized by IMI (Institute for Research and Development Ltd., Haifa, Israel). Peptides were synthesized by the solid phase methodology and supplied at > 95% purity. The correct composition and purity of the peptides were verified by HPLC, mass spectrometry and amino acid analysis. Peptides stock solutions (5 mg/ml in 10% dimethylsulfoxide (DMSO) in H₂O) were kept at -20 °C.

### Isolation and purification of mast cells

Mast cells from the peritoneal cavity of C.R rats were isolated in Tyrode buffer (137 mM NaCl, 2.7 mM KCI, 1 mM MgCl₂, 0.4 mM NaH₂PO₄, 20 mM Hepes, 1.0 mM CaCl₂, 5.6 mM glucose, 1 mg/ml BSA, pH 7.2) and purified over a Ficoll gradient. A suspension of washed peritoneal cells was placed over a cushion of 30% Ficoll 400 (Pharmacia Biotech.) in buffered Saline containing 0.1% BSA, and centrifuged at 150xg for 15 min. The purity of mast cells recovered from the bottom of the tube was > 90%, as assessed by toluidine blue staining.

### Triggering histamine secretion from intact cells

Purified mast cells (duplicated of 10⁵ cells/0.5ml) were incubated in Tyrode buffer with buffer or with desired concentrations of the indicated peptide for 2 h. at 37°C. Histamine secretion was subsequently stimulated by the indicated concentration of compound 48/80 (Sigma) dissolved in Tyrode buffer. Incubation with compound 48/80 was carried out for 20 min. at 37°C. The reaction was terminated by placing the tubes on ice. The cells were sedimented by centrifugation at 150xg for 5 min. and the supernatants were collected. The amount of histamine release was determined as previously described (Aridor et al., 1990). Briefly, cell pellets were lysed using 0.1N NaOH and the volume of each sample was adjusted to 0.5 ml by H₂0. Histamine content was assayed using the o-phtalaldehyde (OPT) fluorimetric method (Shore et al., 1959). Aliquots of 0.4 ml from the supernatants and cell lysates were incubated with 1.6 ml H₂0, 0.4 ml IN NaOH and 0.1 ml of 10 mg/ml OPT in methanol, for 4 min. at room temperature. The reaction was terminated by the addition of 0.2 ml 3N HCl. Samples were centrifuged at 150xg for 5 min. and 0.2 ml samples were transferred to a 96 well plate. The histamine spectrofluorometric assay was run in microplates using a microplates reader (FL-600, Biotek instruments Winooski, VT, USA). Samples were excited by light at 340nm and read at 440nm. Histamine release was calculated as the percentage of total histamine content (supernatant / pellet + supernatant) in each sample. Each data point represents the average of duplicate measurements. The spontaneously released histamine was subtracted. Statistical analysis and plotting were done with Excel^{®} (Microsoft Ltd., Washington, USA).

### Induction of Histamine Secretion by Substance P

The induction of histamine secretion was performed by 50 µM of the physiological basic secretagogue substance P, and thus mimics histamine release in vivo. In these experiments, mast cells were incubated with increasing concentrations of peptide 2 for 2 h. at 37 °C. Following the two hour incubation period, histamine secretion was stimulated by 5 µl of substance-P at a final concentration of 50 µM. Histamine release was determined as previously described, and is presented as percentage of the maximal response, which corresponds to a known percentage of the total cellular histamine content.

### RESULTS

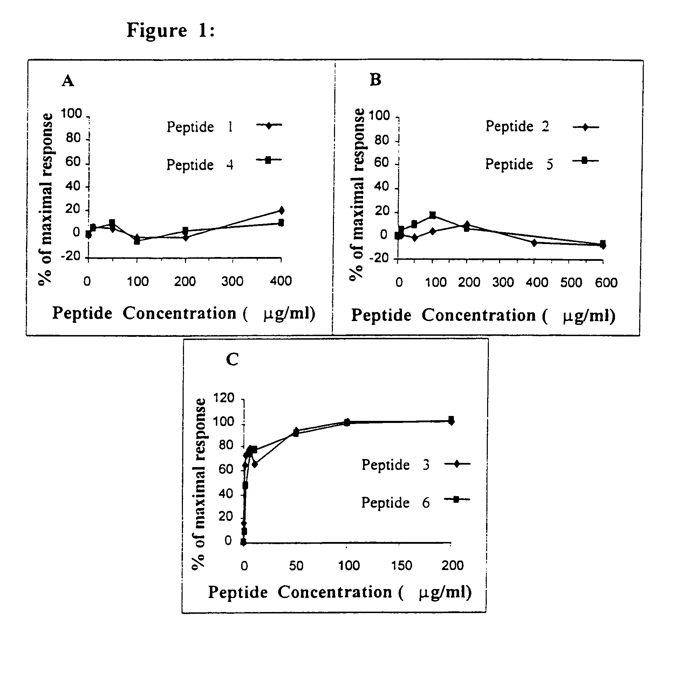

As demonstrated in Figure 1, peptides 1 and 4 (which both include the leader motif of the signal sequence within human integrin β₃ linked to the C-terminal sequences of Gαi₃ or Gαt, respectively) exerted hardly any stimulatory effect on histamine secretion at a concentration range of up to 400 µg/ml of the peptide (Figure 1 A). Similar results were obtained with peptides 2 and 5 (which both include the leader motif of the signal sequence of the Kaposi fibroblast growth factor linked to the C-terminal sequences of Gαi₃ or Gαt, respectively) at a concentration range of up to 600 ug/ml of the peptide (Figure 1B). In contrast, peptides 3 and 6 (which both include the leader motif of the homeodomain of a *Drosophila* transcription factor linked to the C-terminal sequences of Gαi₃ or Gαt, respectively) induced histamine secretion from mast cells in a concentration dependent manner (Figure 1C). These results suggest that peptides containing the h region of a signal peptide sequence of either the signal sequence of the Kaposi fibroblast growth factor or the signal sequence within human integrin β₃, can serve as potential inhibitors of mast calls exocytosis, as they do not exert side effects of effecting histamine secretion. In contrast, peptides including the leader motif of the homeodomain of the *Drosophila* transcription factor induce side effects of histamine secretion, and therefore can not serve as potential inhibitors of mast calls exocytosis.

In order to investigate the ability of peptides 1, 2, 4 and 5, which did not exert side effects on mast cells *in vitro,* to block allergen-induced exocytosis, these peptides were examined for their ability to block compound 48/80 induced histamine secretion from intact mast cells *in vitro.*

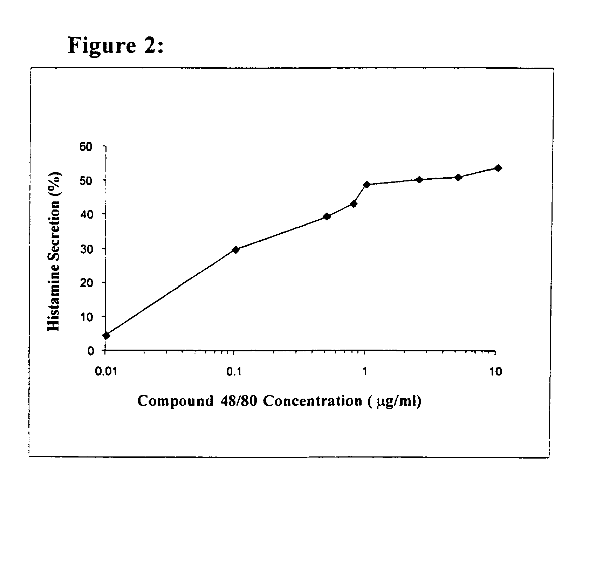

First, the effect of compound 48/80 on exocytosis from intact mast cells was determined. A calibration curve is demonstrated in Figure 2, displaying a dose response of histamine release induced by compound 48/80. Histamine release was determined and is presented as the net secretion, that is % of the total cellular histamine following subtraction of the spontaneously released histamine. Half-maximal release was obtained at 0.1 ug/ml while maximal release was obtained at 1-10 µg/ml. Accordingly, in order to analyze the potential of each peptide to block histamine secretion, histamine secretion was induced with 5 µg/ml compound 48/80, a concentration that induces a maximal histamine release, while the cells were incubated in the presence of increasing concentrations of each peptide.

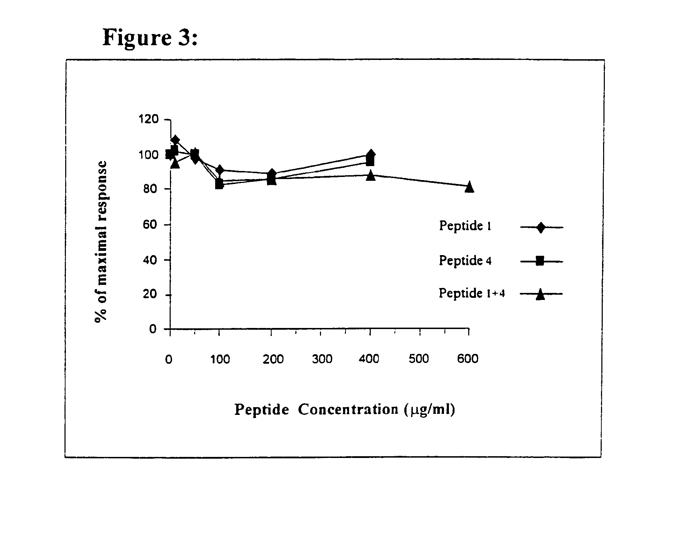

Peptides 1 and 4 (which include the leader motif of the signal sequence within human integrin β₃ and the C-terminal sequences of Gαi₃ or Gαt, respectively) exerted hardly any blocking effect on histamine secretion that was induced by compound 48/80, at a concentration range of up to 400 µg/ml of the peptide (Figure 3). Applying both peptides simultaneously in a concentration range of even up to 600 µg/ml did not reveal any synergistic effect. Thus, peptides 1 or 4, which include the leader motif of the signal sequence within human integrin β₃, although exerting no side effects on mast cells in our *in vitro* system, do not block histamine secretion, and hence cannot serve as potential inhibitors of mast cells exocytosis.

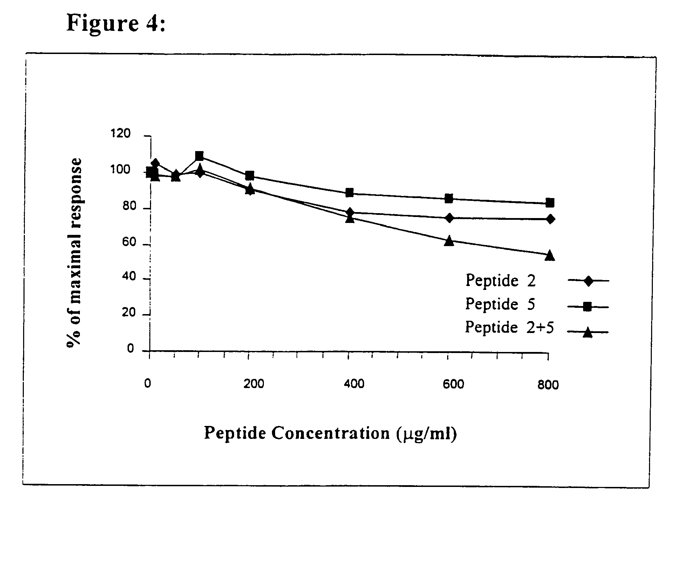

On the other hand, peptides 2 and 5 (which both include the leader motif of the signal sequence of the Kaposi fibroblast growth factor linked to the C-terminal sequences of Gαi₃ or Gαt, respectively) exerted inhibition of histamine secretion that was induced by compound 48/80 (Figure 4). Peptide 5 demonstrated very moderate inhibition of up to 16% of the maximal response at a concentration of 800 µg/ml. Peptide 2 was more potent and inhibited by 25% the maximal response, at a concentration of 600 µg/ml. Applying both peptides simultaneously revealed a synergistic effect causing more extensive inhibition of histamine secretion at a concentration dependent manner (Inhibition of 25 %, 38% and 45% of the maximal response at concentrations of 400, 600 and 800 µg/ml, respectively). Thus, peptides 2 and 5 can serve as potential inhibitors of mast cell exocytosis, while exerting no side effects in this experimental system.

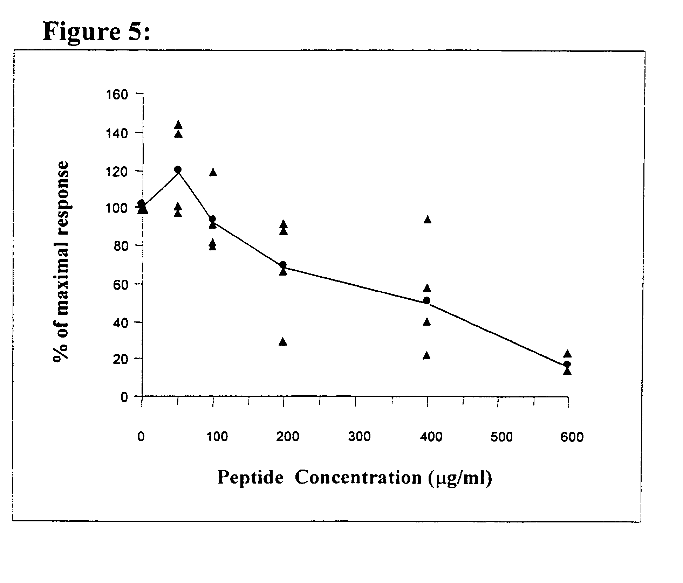

In order to examine further the ability of peptide 2 to serve as a blocker of histamine release, induction of histamine secretion was performed by 0.1 µg/ml compound 48/80, a concentration that was demonstrated previously to cause half-maximal release of histamine (Figure 2), and thus mimics histamine release *in vivo* occurring following exposure to substance-P or other physiological basic secretagogues. As shown in Figure 5, the results demonstrate that peptide 2 exerted inhibition of histamine secretion induced by 0.1 µg/ml of compound 48/80. This inhibition was dose dependent with maximal inhibition of 84% achieved at a concentration of 600 µg/ml peptide.

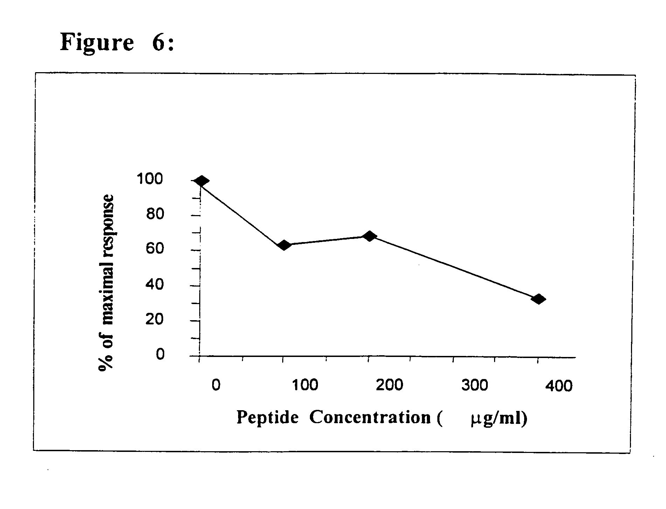

Figure 6 shows that peptide 2 also exerted inhibition of histamine secretion induced by substance P. This inhibition was dose dependent with maximal inhibition of 67% achieved at a concentration of 400 µg/ml peptide. Thus, peptide 2 has the potential to fully block histamine release from mast cells induced by physiological concentrations of basic secretagogues. This peptide may thus provide a unique and effective means to block mast cell exocytosis that leads to the allergic reaction.

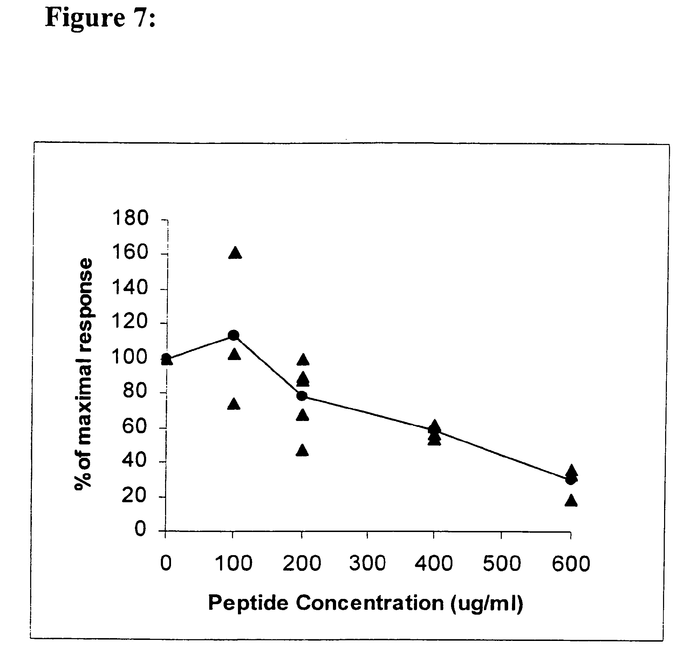

In order to examine further the ability of peptide 5 to serve as a blocker of histamine release, mast cells were incubated with different concentrations of peptide 5, followed by induction of histamine secretion by 0.1 µg/ml compound 48/80. As shown in Figure 7, the results demonstrate that peptide 5 exerted inhibition of histamine secretion induced by 0.1 µg/ml of compound 48/80. This inhibition was dose dependent with maximal inhibition of 70% achieved at a concentration of 600 µg/ml peptide.

### EXAMPLE 2

### PEPTIDE MODIFICATIONS

The results described in Example 1 above demonstrated that both peptide 2 and peptide 5 have the ability to block mast cell degranulation, with peptide 2 demonstrating higher efficacy as compared to peptide 5. Peptide 2 had the highest inhibition of histamine release demonstrated, with 84% inhibition, as opposed to 70% for peptide 5.

Several point mutations and biochemical modifications were performed in each peptide, in order to improve peptide solubility and efficacy, as well as to investigate structure/function relationships.

The first such mutation is a point mutation in peptide 5. Specifically, in peptide 5, the glutamic acid in position 18 was replaced by asparagine, to form peptide 5-modified (Peptide 5m - AAVALLPAVLLALLAPKNNLKDCGLF). In this peptide the last 10 amino acids are homologous to the C-terminal sequence of Gαi₂.

Next, in an attempt to improve peptide solubility, a lysine residue was added to the N-terminus of the peptides 2 and 5, to form 2 new sequences:
Peptide 12: KAAVALLPAVLLALLAPKNNLKDCGLF
Peptide 13: KAAVALLPAVLLALLAPKNNLKECGLY

Amino acids were then deleted, in order to shorten peptides 2 and 5, by removing 3 amino acids from positions 17-19 to form 2 new sequences, respectively:
Peptide 20: AAVALLPAVLLALLAPLKECGLY
Peptide 21: AAVALLPAVLLALLAPLKDCGLF

Also, various point mutations were made in peptide 2. First, cysteine residue was replaced, in an attempt to improve peptide efficacy and to avoid possible oxidation of the peptide. Specifically, the cysteine residue in position 23 of peptide 2 was replaced by serine, to form the following sequence:
Peptide 25: AAVALLPAVLLALLAPKNNLKESGLY

An additional approach to improve peptide solubility involved changing the configuration of the peptide N-terminus to D/L configuration, thus forming the sequence:
Peptide 2 D/L: H- (D, L)-A- (D, L)-A-VALLPAVLLALLAPKNNLKECGLY

Also, in order to improve peptide solubility, a succinyl residue was added to the N-terminus of the peptides, to form 2 new sequences:
Peptide 2-Succinylated(2-Suc):
   Succinyl-AAVALLPAVLLALLAPKNNLKECGLY
Peptide 5-Succinylated (5-Suc):
   Succinyl-AAVALLPAVLLALLAPKENLKDCGLF

All of these peptides were tested as previously described in Example 1.

### RESULTS

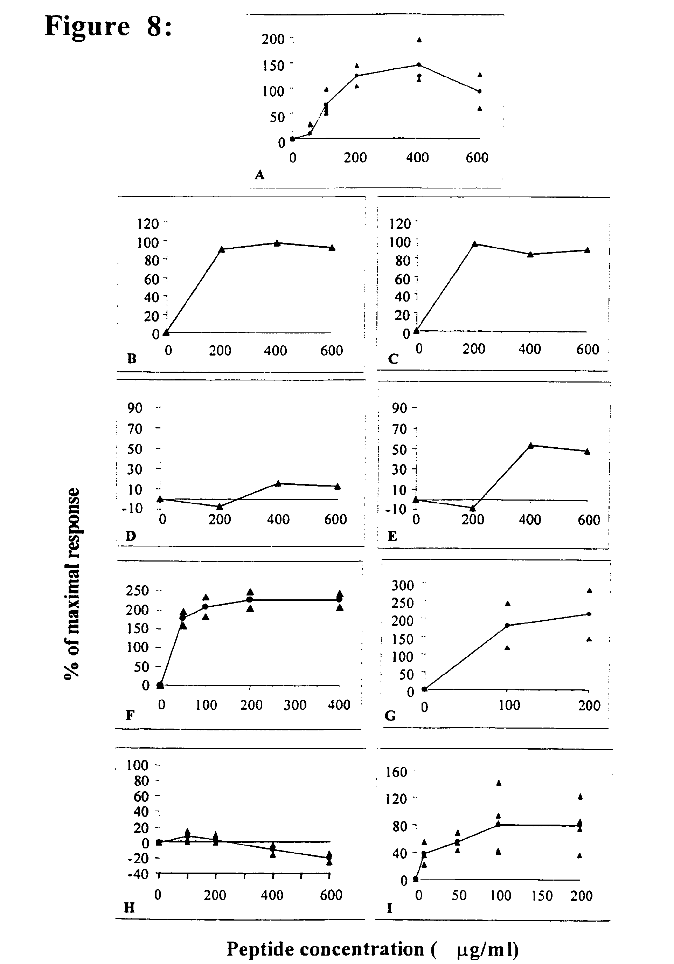

Certain specific modifications of the peptides 2 and 5 (previously exhibiting the desired anti-allergic activity), rather than preventing or abolishing histamine secretion, actually potentiated such secretion, as shown in Figure 8. These peptides include peptide 5m (which contains a homologue sequence to the C-terminus of Gαi_{2;} Figure 8A); peptides 12 and 13 (which contain a lysine residue at the N-terminus of the peptide; Figures 8B,C respectively); peptide 25 (which contains a serine residue at position 23 instead of a cysteine; Figures 8F); peptide 2-D/L (containing an alternative D/L configuration at the N-terminus of the peptide; Figure 8G): and peptide 5-Suc (which contains a succinyl residue at the N-terminus of the peptide; Figure 81). Since these peptides induced histamine secretion from mast cells in a concentration dependent manner, without the presence of any additional allergen, these peptides actually cause allergic side effects and cannot serve as potential inhibitors of mast cell exocytosis.

With regard to the remaining peptides, peptides 20 and 2-Suc did not induce histamine secretion from mast cells (demonstrated in Figure 8D,H, respectively). Peptide 21 induced some histamine secretion at higher doses of 400 and 600 µg/ml (Figure 8E).

In order to investigate the ability of these peptides to block allergen-induced exocytosis, the peptides were examined for their ability to block compound 48/80 induced histamine secretion from intact mast cells *in vitro.* Mast cells were incubated with different concentrations of each peptide, followed by induction of histamine secretion by compound 48/80.

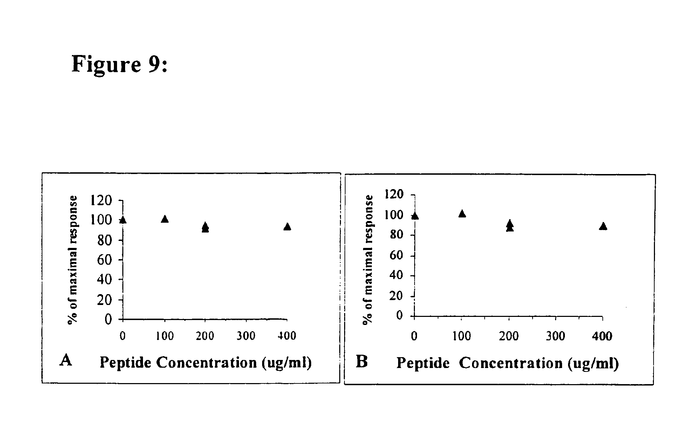

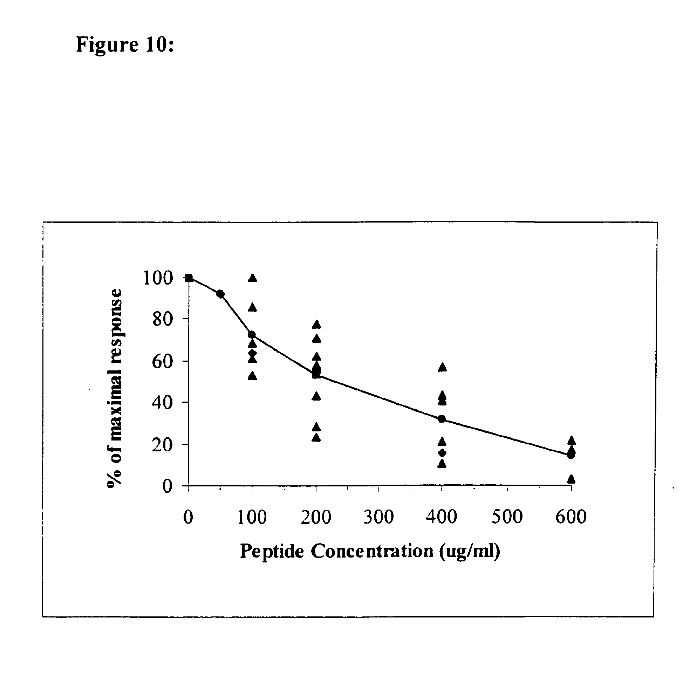

As shown, peptides 20 and 21 did not block histamine secretion as induced by compound 48/80 (Figure 9). These results suggest that deletion of 3 amino acids at positions 17-19 causes the loss of desired activity, and such that these deleted peptides therefore can not serve as potential inhibitors of mast cells exocytosis in these conditions. However, as demonstrated in Figure 10, peptide 2-Suc did block histamine secretion that was induced by compound 48/80. These results illustrate that peptide 2-Suc, which is more soluble in the assay medium, as compared to peptide 2, had no stimulatory effect on mast cells while exerting inhibition of histamine secretion induced by compound 48/80. This inhibition was dose dependent with maximal inhibition of 80% achieved at a concentration of 600 µg/ml peptide.

Thus, these results demonstrate that peptide 2 (which includes the leader motif of the signal sequence of the Kaposi fibroblast growth factor linked to the C-terminal sequences of Gαi₃) is a potent inhibitor of mast cell degranulation, *in vitro.* Addition of a succinyl residue to the N-terminus of this peptide increased both its solubility and its efficacy. Both peptides blocked compound 48/80 induced histamine secretion from mast cells in a dose dependent manner, where maximal inhibition was received at 600 µg/ml. The IC₅₀ decreased from 400 µg/ml (Peptide 2; Figure 5) to 200 µg/ml (Peptide 2-Suc; Figure 10). Without wishing to be limited by a single hypothesis, the apparent increase in potency may be caused by the increased solubility of the succinylated form.

Peptides 2 and 2-Suc were also tested *in vitro* for their ability to block histamine secretion from rat peritoneal mast cells, in response to the IgE-dependent mechanism. The IgE dependent pathway is activated in response to an immunological trigger, brought about by aggregation of the high affinity receptors (F_{c}εRI) for IgE, which are present on the cell surface of mast cells. This response involves crosslinking of cell bound IgE antibodies by the corresponding antigens (allergens).

Isolated, purified mast cells were sensitized in the presence of a monoclonal DNP-specific IgE antibody (1µg/10⁶ cells) for 1 hour at 37°C. The cells were washed 3 times and incubated for 2 hours with different concentrations of each peptide, followed by triggering with the antigen DNP-BSA (100 ng/ml) in the presence of Brain Extract (0.1mg/ml), for 20 min. at 37°C. Placing the tubes in ice terminated the reaction. The amount of histamine released from the cells was monitored.

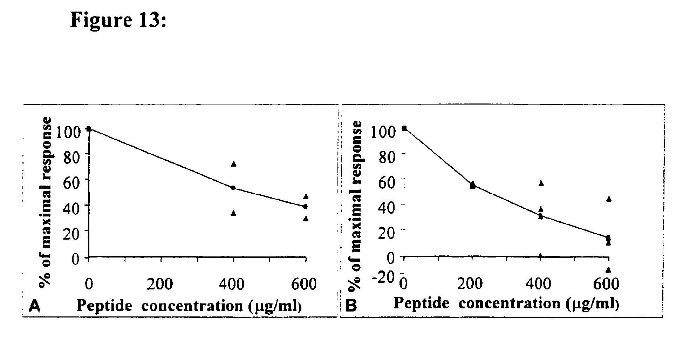

The results demonstrate that both peptides -2 and 2-Suc effectively block histamine secretion from isolated and purified rat mast, activated by an immunological trigger. Peptide 2-Suc, as a presumably more soluble version of peptide 2, is more effective as an inhibitor of histamine secretion, demonstrating 90% inhibition at a concentration of 600 µg/ml (Figure 13).

### EXAMPLE 3

### PEPTIDE CYCLIZATION

Based on the results of Examples I and 2, similar peptide sequences, differing only in one or two amino acids, may be significantly distinct from each other in their activity and the response they induce in mast cells.

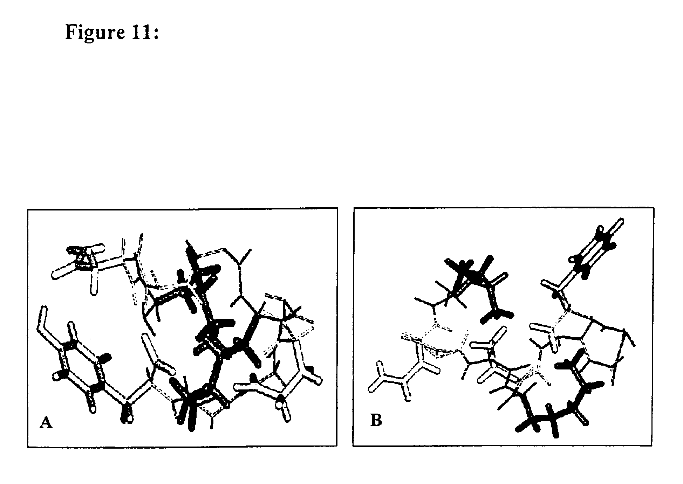

In order to establish possible structure/function relationships, and to demonstrate the 3D structure of the active sequence of the molecule, as compared to less active sequences, computerized modeling was performed on the C-terminus of peptides, containing different amino acid sequences that demonstrate various levels of activity. The results illustrate a favored cyclic structure (by energy requirements, assuming hydrophobic or hydrophilic environment) of the C-terminus of Peptide 2, as compared to an open structure of peptide 5m, which induces side effects of histamine secretion from the cells (Figure 11).

In light of the aforementioned results, a cyclic form of peptide 2 was synthesized, forming a cyclization between the side chain of Lysine at position 17 and the C-terminus of the peptide:

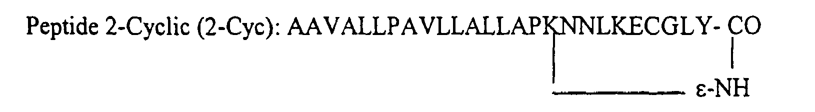

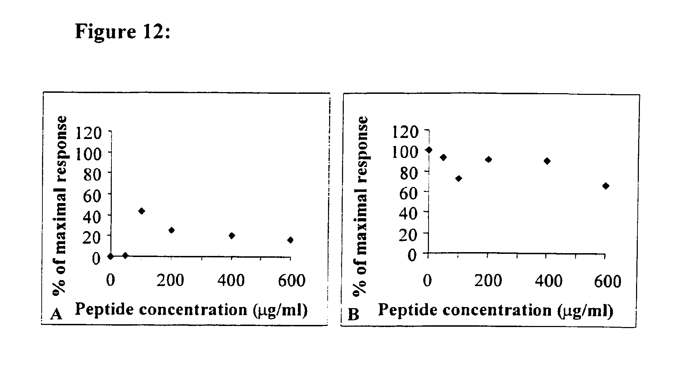

The results demonstrate that peptide 2-Cyc exerted only minor side effects of histamine secretion in mast cells *in vitro,* (at a concentration of 100 µg/ml). Yet, peptide 2-Cyc demonstrated only limited potential to block compound 48/80 induced histamine secretion, and only at very high peptide concentrations (see figure 12). However, the cyclic peptide also had a very poor solubility in the buffer (Tyrode) used in the assay. Therefore, the low potency may be related to its low solubility.

Table 1 summarizes the results obtained in the *in vitro* system, demonstrating different responses of mast cells to the various peptides.

**Table 1: Summary of in vitro results - histamine secretion from isolated mast cells, following treatment with different peptides**

| **Peptide** | **Sequence** | **Secretagogue*** | **Inhibitor **** | **Remarks** |
|---|---|---|---|---|
| 2 | AAVALLPALLALLAPKNNKEGGLY | - | ++ | Intermediate solubility |
| 2-Suc | Succinyl- AAVALLPAVLLALLAPKNNLKECGLY | - | +++ | Good solubility |
| 2-Cyc | AAVALLPAVLLALLAPKNNLKECGLY ε-NH | -/+ | + | Poor solubility |
| 5 | AAVALLPAVLLALLAPKENLKDCGLF | - | ++ | Intermediate |
| 5m | AAVALLPAVLLALLAPKEnNLKDCGLF | + | - | |
| 5-Suc | Succinyl- AAVALLPAVLLALLAPKENLKDCGLF | + | - | |
| 12 | KAAVALLPAVLLALLAPKNNLKDCGLF | + | - | |
| 13 | AAVALLPALLALLAPKNNKEGGLY | + | - | |
| 20 | AAVALLPAVLLALLAPLKEGGLY | - | - | |
| 21 | AAVALLPAVLLALLAPLKDCGLF | -/+ | - | |
| 25 | AAVALLPAVLLALLAPKNNLKESGLY | + | - | |
| 2 D/L | H- (D, L)-A- (D, L)-A- VALLPAVLLALLAPKNNLKECGLY | + | - | |

| | | | | |
|---|---|---|---|---|
| * Histamine secretion following incubation of mast cell with different concentrations of each peptide: - No side effect of histamine secretion. + Peptide that induce histamine secretion (Secretagogue). * * Degree of Inhibition of histamine secretion from mast cells, followed by incubation with different concentrations of each peptide and induction of the allergic reaction. +++ Potent inhibitor (>85% inhibition), ++ Moderate inhibitor (> 70% inhibition), + Poor inhibitor (< 50% inhibition), - No inhibition. | | | | |

### EXAMPLE 4

### TESTING OF THE EFFECTS OF THE TREATMENT OF THE PRESENT INVENTION IN VIVO

The ability of various peptides to block the release of histamine secretion *in vivo* was tested on the skin of rats by using compound 48/80 as the allergen. Peptide 2, and the succinylated derivative thereof, were shown to effectively block the allergic response by preventing the release of histamine from mast cells *in vivo.* The experimental method was as follows.

### MATERIALS AND METHODS

The hair of the abdominal area of C.R rats was carefully removed with an electric clipper and a depilatory cream. In each animal the abdominal area was divided to six equal zones that were marked by pen. Each zone was either subjected to peptide treatment or served as a control. In the first set of experiments, the peptide was topically applied as follows: 36 µl of peptide solution at the indicated concentration (dissolved in 72% DMSO in saline) was applied on desired abdominal area. In the second set of experiments, the peptide was injected intradermally as follows: 20 µl of peptide solution at different concentrations (dissolved in 10% DMSO in saline) was injected intradermally to an indicated abdominal area using a 27-gauge sterile needle.

Skin tests were performed 0.5, 1 or 2 hours following application of the peptide. Skin tests were performed by injecting intradermally 20 µl of the allergen (0.1 mg/ml compound 48/80 dissolved in saline) or saline alone, into the center of each marked area on the abdominal skin using a 27-gauge sterile needle. The allergic response was monitored by outlining with a marker the wheals which developed in response to allergen or saline treatment.

To quantitate the skin test results, the marker signs were transferred onto paper with scotch tape. The areas of the wheals were outlined and calculated by a computerized planimeter (Hewlett-Packard), as previously described (Sussman et al., 1982).

### RESULTS

The area of the wheals which developed in response to topical application of peptide 2 followed by compound 48/80 or saline injection is given in Table 2, with the net wheal area given in Table 3. These results demonstrate that the size of the wheals which developed after saline injection range between 72-93 mm² while the size of the wheals which developed after the injection of compound 48/80 range between 114-134 mm², demonstrating a significant allergic response induced by intradermal injection of compound 48/80 as compared to saline. The wheals which developed following the application of peptide 2 and saline injection, without compound 48/80, were slightly smaller than the wheals developed following no peptide application (Table 3A). These results demonstrate that topical application of peptide 2 by itself exerts no stimulatory effect on the cutaneous allergic reactions.

Comparing the net allergic reaction, which is the wheal area induced by saline injection subtracted from the wheal area induced by the injection of compound 48/80 injection, reveal that topical application of 350 micrograms of peptide 2 reduced compound 48/80-induced allergic reaction from a net wheal area of 41-42 mm² to a net wheal area of 9-12 mm² (Table 3B).

These results further reinforce the *in vitro* results, demonstrating that peptide 2 has the potential to also block allergic reactions *in vivo*, such as the cutaneous allergic reactions. The area of the wheals which developed in response to intradermal injection of peptide 2 followed by compound 48/80 or saline injection is given in Table 4, with the net wheal area given in Table 5. The wheals, which developed following the injection of peptide 2 and saline, without compound 48/80, were similar to the wheals developed following no peptide application (Table 4, 5A). These results demonstrate that intradermal injection of peptide 2 by itself exerts hardly any stimulatory effect on the cutaneous allergic reactions.

Comparing the net allergic reaction, which is the wheal area induced by saline injection subtracted from the wheal area induced by the injection of compound 48/80, reveal that intradermal injection of 20 µg of peptide 2 already reduced compound 48/80-induced allergic reaction (Table 5B), while a higher dose of 200 µg of the peptide, increased the inhibition effect (Table 5B).

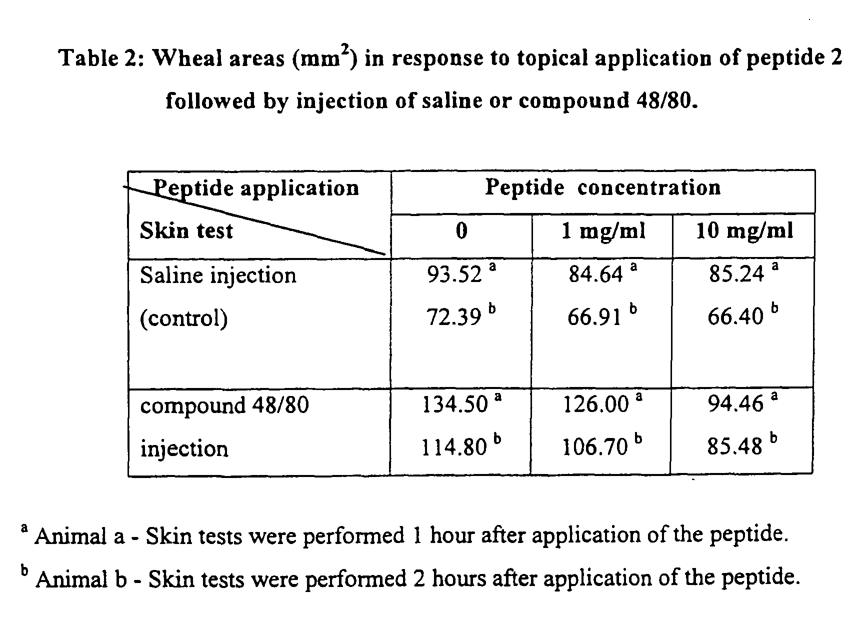

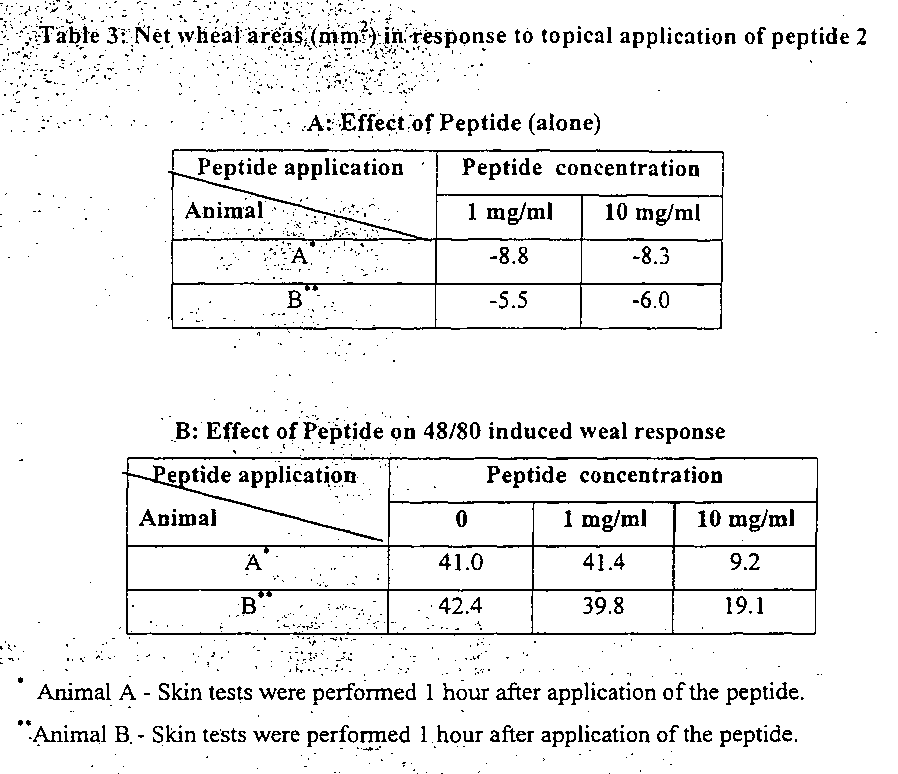

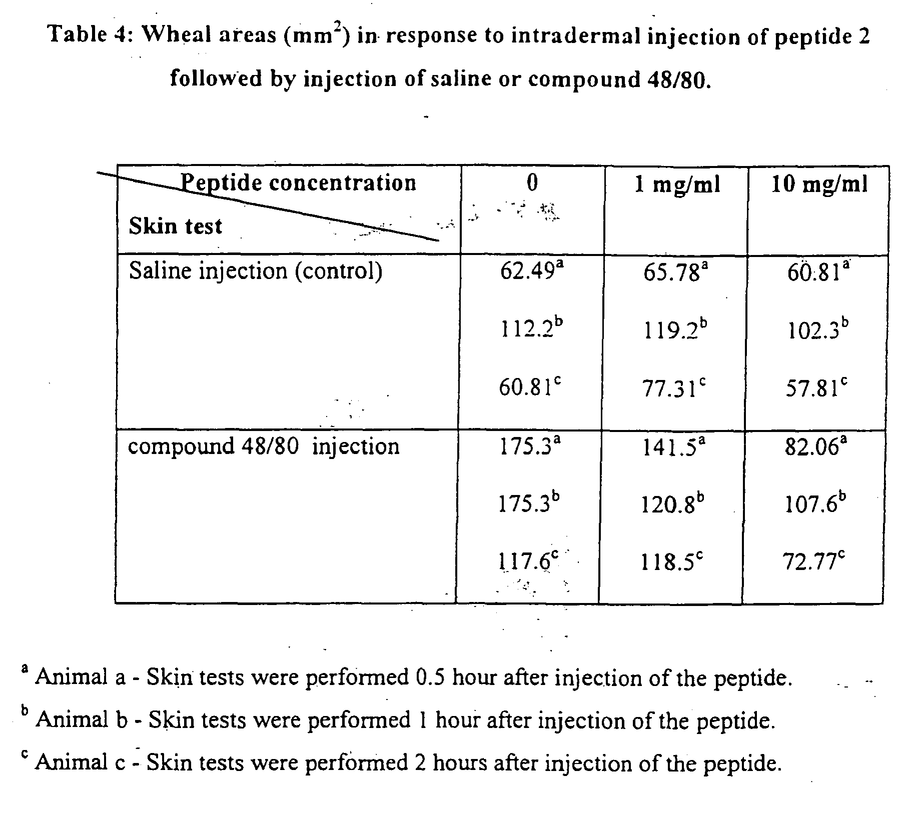

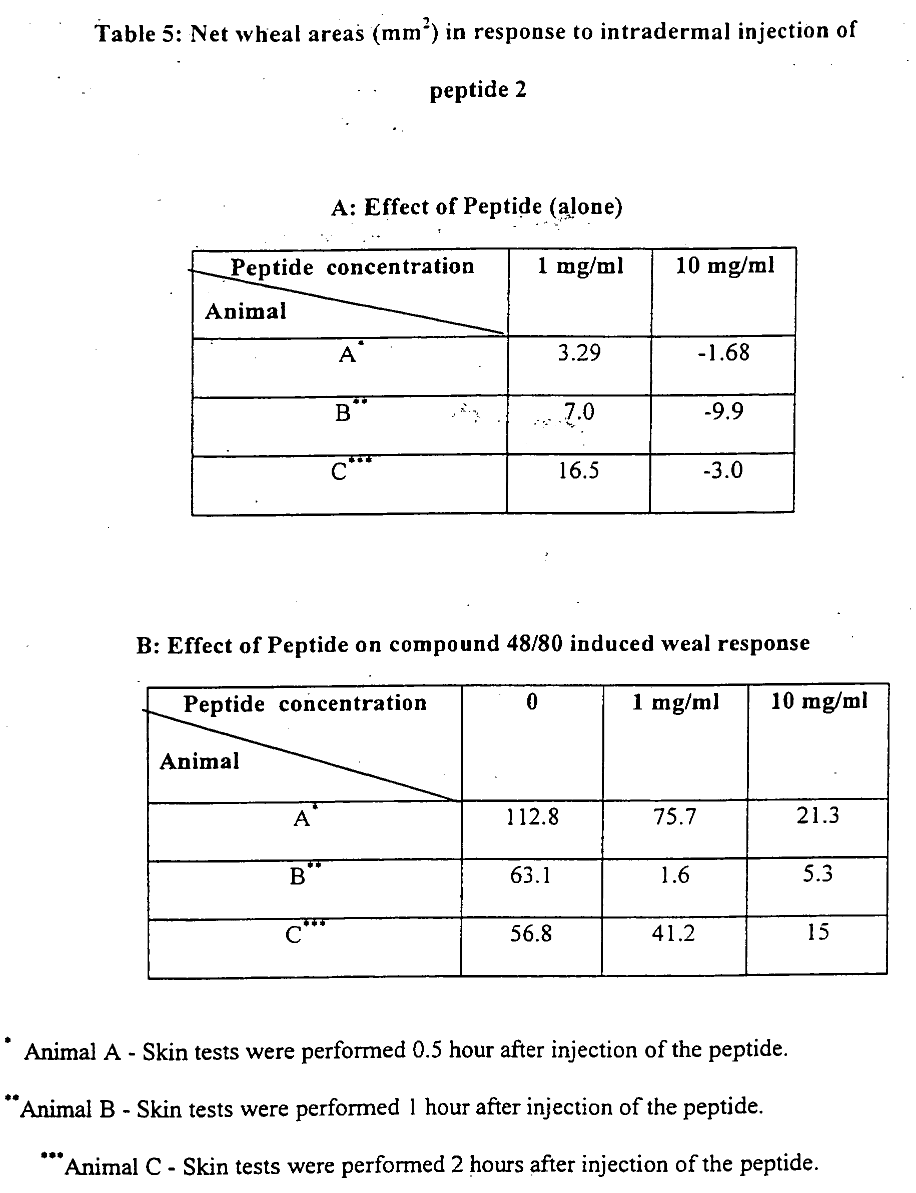

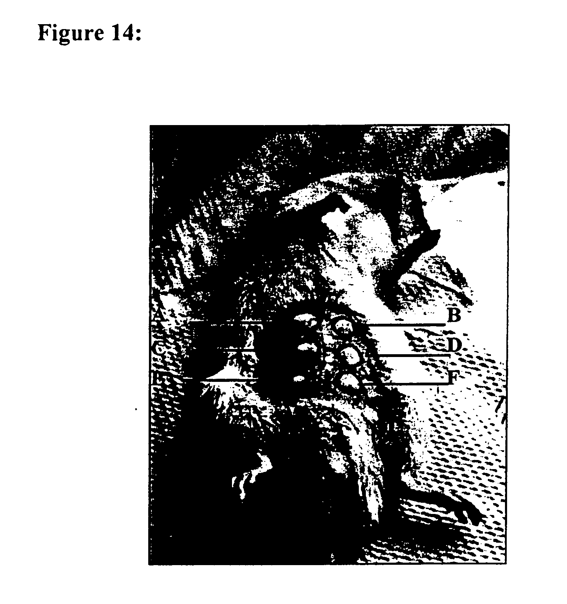

Additional skin tests were performed on rats, using compound 48/80 as the allergen, to test the ability of peptide 2, peptide 2-Suc, and peptide 2-Cyc, to block allergic reactions *in vivo*. The abdominal skin of the rats was subjected to peptide or vehicle treatment (intradermal injection). The allergic response was then induced by intradermal injection of compound 48/80 (0.1 mg/ml) at various times following the application of the peptide. A representative experiment is demonstrated in Figure 14. Calculating the areas of the wheals, which developed, quantitated the allergic response

Table 6 presents the mean areas of the wheals, which developed in response to intradermal injection of peptide 2, followed by either compound 48/80 or saline injection applied after 0.5h. or 1h. These results demonstrate that intradermal injection of peptide 2 has the potential to block compound 48/80 induced allergic reaction *in vivo.* Peptide 2 reduced the wheal area in a dose dependent manner, reaching significant inhibition at doses of 20 and 200 µg peptide (injection of 20 µl from a stock solution of 1 mg/ml or 10 mg/ml). Significant inhibition was detected at different timing (1 hour or 2 hours before the allergic induction; Wheal areas differ significantly from the control group, P< 0.01, student's T-test).

Table 7 presents the mean areas of the wheals, which developed in response to intradermal injection of peptide 2-Suc, followed by either compound 48/80 or saline injection applied after 0.5h. or 1h. These results demonstrate that intradermal injection of peptide 2-Suc blocks the allergic reaction induced by compound 48/80 *in vivo*. However peptide 2 Suc was less effective than peptide 2 in the *in vivo* system.

Peptide 2-Suc significantly reduced the wheal when applied 0.5 hour before the allergic reaction, at a dose of 200 µg peptide. A lower dose or longer timing (1 hour before the allergic induction) had no effect.

Table 8 presents the mean areas of the wheals, which developed in response to intradermal injection of peptide 2-Cyc, followed by either compound 48/80 or saline injection applied after 0.5h. or 1h. These results demonstrate that intradermal injection of peptide 2-Cyc blocks the allergic reaction induced by compound 48/80 in *vivo*. However, peptide 2-Cyc is also less effective than peptide 2 in the *in vivo* system, demonstrating a significant decrease in wheal area when applied 0.5 hour before the allergic induction, at a dose of 200 µg peptide. A lower dose or longer timing (1 hour before the allergic induction) had no effect.

Noteworthy, intradermal injection of each peptide alone, exerted no stimulatory effect on the cutaneous allergic reactions, thus indicating that each compound by itself is not allergic (see Tables 5, 6 and 7).

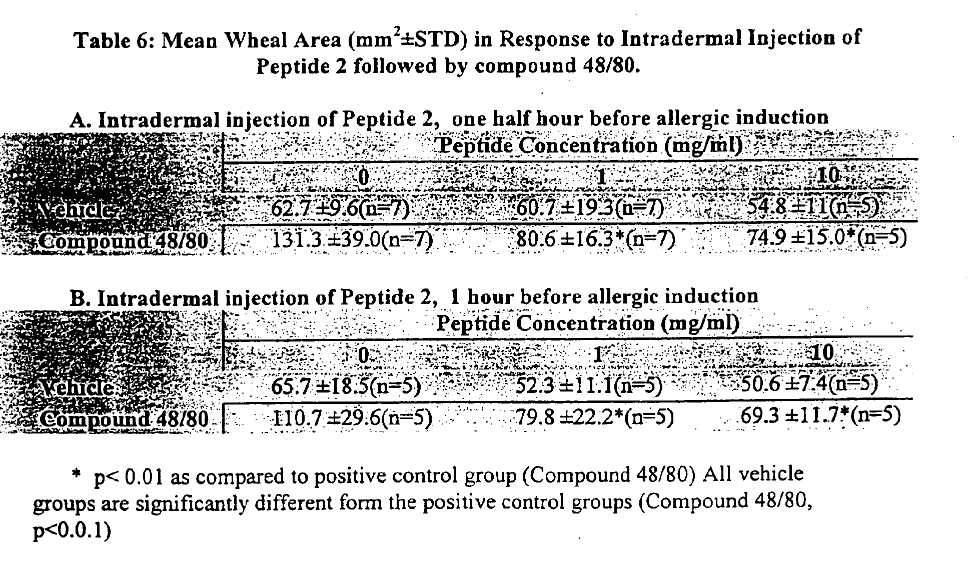

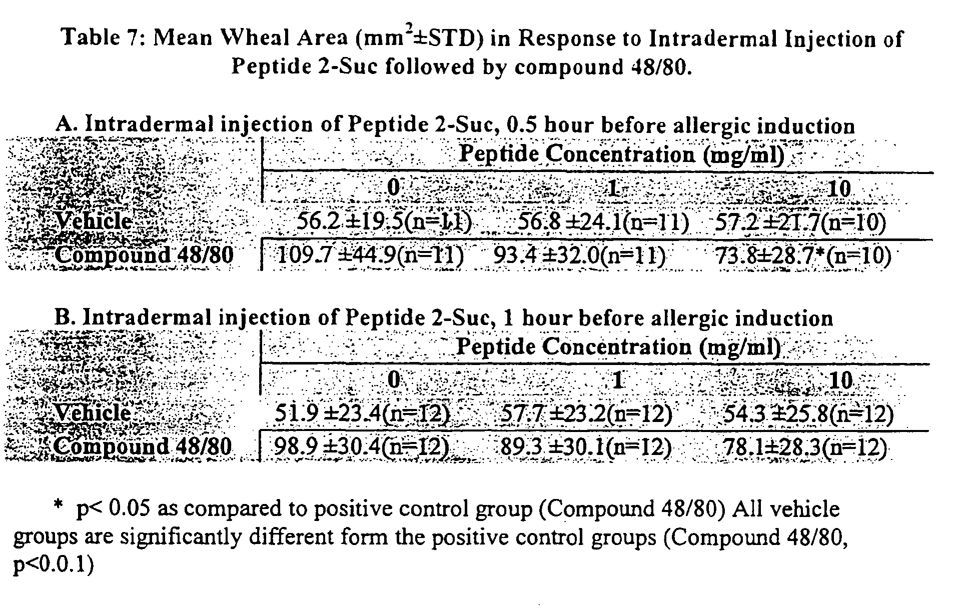

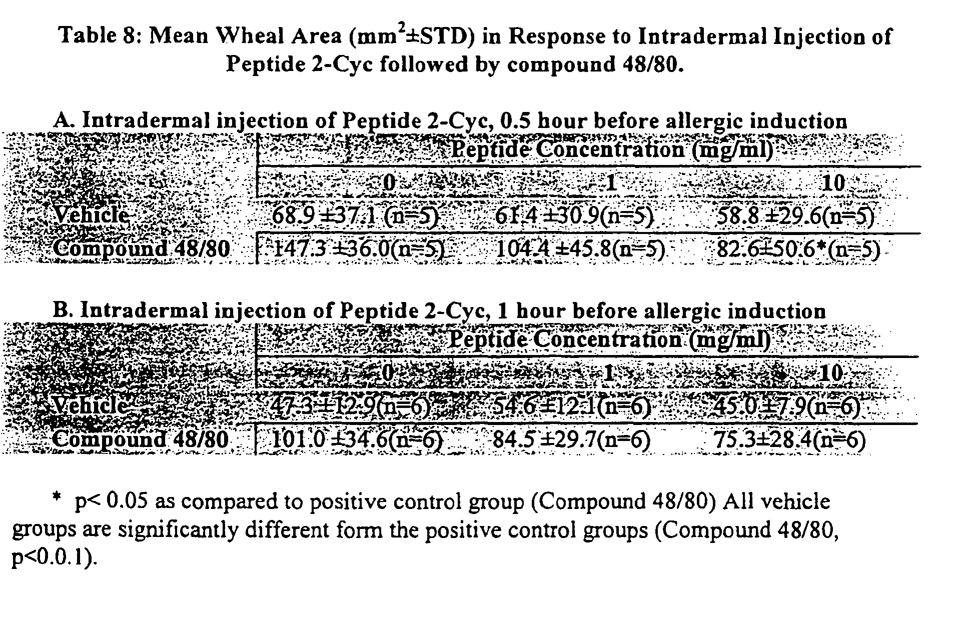

### EXAMPLE 5

### METHODS AND COMPOSITIONS FOR ADMINISTRATION

The peptides of the present invention, and their homologues or related compounds, hereinafter referred to as the "therapeutic agents of the present invention", can be administered to a subject by various ways, which are well known in the art. Hereinafter, the term "therapeutic agent" includes a peptide as previously defined, in particular peptide 2 and/or homologues, analogues or mimetics thereof, such as the succinylated derivative thereof, or any biologically active substance having a substantially similar effect as previously defined.

Hereinafter, the term "subject" refers to the human or lower animal to whom the therapeutic agent is administered. For example, administration may be done topically (including opthalmically, vaginally, rectally, intranasally and by inhalation), orally, or parenterally, for example by intravenous drip or intraperitoneal, subcutaneous, or intramuscular injection.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include but are not limited to sterile aqueous solutions which may also contain buffers, diluents and other suitable additives.

Dosing is dependent on the severity of the symptoms and on the responsiveness of the subject to the therapeutic agent. Persons of ordinary skill in the art can easily determine optimum dosages, dosing methodologies and repetition rates.

### EXAMPLE 6

### METHOD OF TREATMENT OF ALLERGIC CONDITIONS

As noted above, the therapeutic agents of the present invention have been shown to be effective inhibitors of the allergic process by blocking mast cell degranulation, thereby preventing and/or alleviating an allergic condition. The following example is an illustration only of a method of treating an allergic condition with the therapeutic agent of the present invention, and is not intended to be limiting.

The method includes the step of administering a therapeutic agent, in a pharmaceutically acceptable carrier as described in Example 3 above, to a subject to be treated. The therapeutic agent is administered according to an effective dosing methodology, preferably until a predefined endpoint is reached, such as the absence of a symptom of the allergic condition in the subject, or the prevention of the appearance of such a symptom in the subject.

Allergic conditions for which the therapeutic agents of the present invention are useful include, but are not limited to, nasal allergy, irritation or allergic reactions in the eyes, allergic reactions in the skin including any type of allergen-induced rash or other skin irritation or inflammation, acute urticaria, psoriasis, psychogenic or allergic asthma, interstitial cystitis, bowel diseases, migraines, and auto-immune diseases such as multiple sclerosis.

### EXAMPLE 7

### METHODS FOR MANUFACTURING THE THERAPEUTIC COMPLEX OF THE PRESENT INVENTION

The therapeutic complex of the present invention can be manufactured in various ways. For example, if the therapeutic complex includes a peptide for at least one the first segment and the second segment, or if the entire therapeutic complex is a peptide, then such a peptide could be manufactured by peptide synthetic methods which are well known in the art.

Alternatively, such a peptide could be produced by linking the signal sequence and the biologically active moiety through laboratory techniques for molecular biology which are well known in the art.

By way of illustration a recombinant fusion protein could be prepared which would feature the peptide permeabilization sequence in the N-terminus and the C-terminal moiety of Gαᵢₜ or Gαᵢ₃, preferably including the last 10 amino acids, for production in bacteria. For this purpose, DNA sequences coding for the desired peptides are amplified by PCR and purified. After sequence verification, these DNA sequences are ligated and cloned in an appropriate vector. The resulting recombinant plasmid is expressed in E. coli and the recombinant proteins purified from bacterial extracts.

### References

Aridor M., Traub L.M. and Sagi-Eisenberg R. (1990).Exocytosis in mast cells by basic secretagogues: Evidence for direct activation of GTP-binding proteins. J. Cell Biol. 111:909-917.
Aridor M. and Sagi-Eisenberg R. (1990).Neomycin is a potent secretagogue of mast cells that directly activates a GTP-binding protein involved in. J. Cell Biol. 111:2885-2891.
Aridor M., Rajmilevich G., Beaven M.A. and Sagi-Eisenberg R. (1993). Activation of exocytosis by the heteroptrimeric G protein Gi3. Science 262:1569-1572.
Bienenstock J., Tomioka M., Stead R., Ernst P., Jordana M., Gauldie J., Dolovich J. and Denburg J. (1987). Mast cell involvement in various inflammatory processes. Am. Rev. Respir. Dis. 135:S5-S8.
Chahdi A., Mousli M. and Landry Y. (1998). Substance P-related inhibitors of mast cell exocytosis act on G-proteins or on the cell surface. Eur. J. Pharmacol. 341,329-335.
Columbo M., Horowitz E.M., Kagey-Sobotka A. and Lichtenstein L.M. (1996). Substance P activates the release of histamine from human skin mast cells through a pertussis toxin-sensitive and protein kinase C-dependent mechanism. Clin. Immunol. Immunop.81,68-73.
Devillier P., Regoli D., Asseraf A., Descours B., Marsac J. and Rerioux M. (1986). Histamine release and local responses of rat and human skin to substance P and other mammalian tachykinins. Pharmacology 32:340-347.
Emadi-Khiav B., Mousli M., Bronner C. and Landry Y. (1995). Human and rat cutaneous mast cells: involvement of a G protein in the response to peptidergic stimuli. Eur. J. Pharmacol. 272,97-102.
Ennis M.., Pearce F.L. and Weston P.M. (1980). Some studies on the release of histamine from mast cells stimulated with polylysine.Br. J. Pharmacol. 70:329-334.
Foreman J.C. (1987a). Neuropeptides and the pathogenesis of allergy. Allergy42:1-11.
Foreman J.C. (1987b). Substance P and calcitonin gene-related peptide: Effect on mast cells and in human skin. Int. Archs. Allergy appl. Immun. 82:366-371.
Goldberg A., Korzets Z., Bernheim J. And Mekori Y.A. (1991). Cutaneous responses to histamine, compound 48/80 and codeine in patients with chronic renal failure. Annals Allergy 67 :525-528.
Gomperts B.D., Churcher Y., Koffer A., Lillie T.H.W., Tatham P.E.R. and Whalley T. D (1991). Intracellular mechanisms regulating exocytotic secretion in mast cells. Int. Arch. Allergy Appl. Immunol. 94:38-46.
Hawiger J. 1997. Cellular import of functional peptides to block intracellular signaling. Curr. Opin. Immunol. 9: 189-194.
Kivity S., Sneh E., Greif J., Topilsky M. and Mekori Y.A. (1988). The effect of food and exercise on the skin response to compound 48/80 in patients with food-associated exercise-induced utricaria-angioedema. J. Allergy Clin. Immunol. 81:1155-1158.
Lichtenstein L.M (1993). Allergy and the immune system. Scientific American 269: 116-124.
Lin Y.Z., Yau S.Y., Veach R.A., Torgerson T.R and Hawiger J. 1995. Inhibition of nuclear translocation of transcription factor NF-kB by a synthetic peptide containing a cell membrane-permeable motif and nuclear localization sequence. J.Biol.Chem. 270:14255-14258.
Mousli M., Hugli T.E. Landry Y. and Bronner C. (1994). Peptidergic pathway in human skin and rat peritoneal mast cell activation. Immunopharmacol. 27:1-11.
Pearce F.L., Kassessinoff T.A., Liu W.L. (1989). Characteristics of histamine secretion induced by neuropeptides: Implications for the relevance of peptide-mast cell interactions in allergy and inflammation. Int. Arch. Allergy Appl. Immunol. 88:129-131.
Prochiantz A. 1996. Getting hydrophilic compounds into cells: lessons from homeopeptides. Curr. Opin. Neurobiol. 6:629-634.
Sagi-Eisenberg R. (1993). Signal-transmission pathways in mast cell exocytosis. In: Immunopharmacology of mast cells and Basophils.
Sussman G.L., Harvey R.P. and Schocket A.L (1982). Evaluation of skin test response using two techniques of measurement. Ann. Allergy 48:75-77.
Theoharides T.C. (1996). The mast cell: a neuroimmunoendocrine master player. Int. J. Tissue React. 18:1-21.

## Claims

1. A therapeutic agent for treating an allergic condition comprising an anti-allergic complex molecule, having at least a first peptide having an amino acid sequence AAVALLPAVLLALLAP competent for importation of said molecule into mast cells in vivo, and a second peptide having an amino acid sequence KNNLKECGLY for having an anti-allergic effect within said mast cells, said first peptide being joined to said second peptide via a peptide bond, whereby the complex molecule is capable of exerting its anti-allergic effect in vivo.

2. A therapeutic agent for treating an allergic condition comprising an anti-allergic complex molecule, having at least a first peptide having an amino acid sequence AAVALLPAVLLALLAP competent for importation of said molecule into mast cells in vivo, and a second peptide having an amino acid sequence KENLKDCGLF for having an anti-allergic effect within said mast cells, said first peptide being joined to said second peptide via a peptide bond, whereby the complex molecule is capable of exerting its anti-allergic effect in vivo.

3. The therapeutic agent of claim 2, wherein said complex molecule is a peptide having an amino acid sequence AAVALLPAVLLALLAPKENLKDCGLF and cyclic derivatives thereof.

4. The therapeutic agent of claim 1, wherein said therapeutic agent further comprises a peptide having an amino acid sequence AAVALLPAVLLALLAPKNNLKECGLY.

5. The therapeutic agent of claim 1, wherein said complex molecule is a peptide having an amino acid sequence AAVALLPAVLLALLAPKNNLKECGLY comprising a cyclization between lysine at position 17 and the C terminus of the peptide.

6. The therapeutic agent of claim 1, wherein said complex molecule is a peptide having an amino acid sequence Succinyl-AAVALLPAVLLALLAPKNNLKECGLY.

7. The therapeutic agent of claims 1 to 6, further comprising a pharmaceutically acceptable diluent or carrier.

8. The therapeutic agent of claims 1 to 7, wherein said therapeutic agent is in a dosage form suitable for topical administration to the eye, the skin or to the mucous membrane of a subject, or in a dosage form suitable for administration by inhalation or intranasal administration or in a dosage form suitable for oral or parenteral systemic administration.

## Patentansprüche

1. Therapeutikum zur Behandlung eines allergischen Zustandes umfassend ein anti-allergisches Komplexmolekül, das mindestens ein erstes Peptid mit einer Aminosäuresequenz AAVALLPAVLLALLAP, die kompetent für den Import des Moleküls in Mastzellen *in vivo* ist, und ein zweites Peptid mit einer Aminosäuresequenz KNNLKECGLY zum Verursachen eines anti-allergischen Effekts innerhalb der Mastzellen aufweist, wobei das erste Peptid mit dem zweiten Peptid mittels einer Peptidbindung verbunden ist, wodurch das Komplexmolekül fähig ist, seine anti-allergische Wirkung *in vivo* auszuüben.

2. Therapeutikum zur Behandlung eines allergischen Zustandes umfassend ein anti-allergisches Komplexmolekül, das mindestens ein erstes Peptid mit einer Aminosäuresequenz AAVALLPAVLLALLAP, die kompetent für den Import des Moleküls in Mastzellen *in vivo* ist, und ein zweites Peptid mit einer Aminosäuresequenz KENLKDCGLF zum Verursachen eines anti-allergischen Effekts innerhalb der Mastzellen aufweist, wobei das erste Peptid mit dem zweiten Peptid mittels einer Peptidbindung verbunden ist, wodurch das Komplexmolekül fähig ist, seine anti-allergische Wirkung *in vivo* auszuüben.

3. Therapeutikum nach Anspruch 2, wobei das Komplexmolekül ein Peptid mit der Aminosäuresequenz AAVALLPAVLLALLAPKENLKDCGLF und zyklische Derivate davon ist.

4. Therapeutikum nach Anspruch 1, wobei das Therapeutikum ferner ein Peptid mit der Aminosäuresequenz AAVALLPAVLLALLAPKNNLKECGLY umfasst.

5. Therapeutikum nach Anspruch 1, wobei das Komplexmolekül ein Peptid mit einer Aminosäuresequenz AAVALLPAVLLALLAPKNNLKECGLY ist, umfassend eine Zyklisierung zwischen Lysin an Position 17 und dem C-Terminus des Peptids.

6. Therapeutikum nach Anspruch 1, wobei das Komplexmolekül ein Peptid mit der Aminosäuresequenz Succinyl-AAVALLPAVLLALLAPKNNLKECGLY ist.

7. Therapeutikum nach den Ansprüchen 1 bis 6, ferner umfassend ein pharmazeutisch verträgliches Verdünnungsmittel oder Trägermaterial.

8. Therapeutikum nach den Ansprüchen 1 bis 7, wobei das Therapeutikum in einer Darreichungsform vorliegt, die zur topischen Verabreichung ins Auge, auf die Haut oder die Schleimhaut eines Individuums geeignet ist, oder in einer Darreichungsform, die zur Verabreichung durch Inhalation oder zur intranasalen Verabreichung geeignet ist, oder in einer Darreichungsform, die zur oralen oder parenteralen systemischen Verabreichung geeignet ist.

## Revendications

1. Agent thérapeutique destiné au traitement d'une condition allergique qui comprend une molécule complexe anti-allergique, ayant au moins un premier peptide ayant une séquence d'acides aminés AAVALLPAVLLALLAP compétente pour l'importation de ladite molécule dans les mastocytes in vivo et un deuxième peptide ayant une séquence d'acides aminés KNNLKECGLY pour avoir un effet anti-allergique à l'intérieur desdits mastocytes, ledit premier peptide étant joint audit deuxième peptide par l'intermédiaire d'une liaison peptidique, moyennant quoi la molécule complexe est capable d'exercer son effet anti-allergique in vivo.

2. Agent thérapeutique destiné au traitement d'une condition allergique qui comprend une molécule complexe anti-allergique, ayant au moins un premier peptide ayant une séquence d'acides aminés AAVALLPAVLLALLAP compétente pour l'importation de ladite molécule dans les mastocytes in vivo et un deuxième peptide ayant une séquence d'acides aminés KENLKDCGLF pour avoir un effet anti-allergique à l'intérieur desdits mastocytes, ledit premier peptide étant joint audit deuxième peptide par l'intermédiaire d'une liaison peptidique, moyennant quoi la molécule complexe est capable d'exercer son effet anti-allergique in vivo.

3. Agent thérapeutique selon la revendication 2, dans lequel ladite molécule complexe est un peptide ayant une séquence d'acides aminés AAVALLPAVLLALLAPKENLKDCGLF et ses dérivés cycliques.

4. Agent thérapeutique selon la revendication 1, dans lequel ledit agent thérapeutique comprend en outre un peptide ayant une séquence d'acides aminés AAVALLPAVLLALLAPKNNLKECGLY.

5. Agent thérapeutique selon la revendication 1, dans lequel ladite molécule complexe est un peptide ayant une séquence d'acides aminés AAVALLPAVLLALLAPKNNLKECGLY comprenant une cyclisation entre la lysine en position 17 et l'extrémité C terminale du peptide.

6. Agent thérapeutique selon la revendication 1, dans lequel ladite molécule complexe est un peptide ayant une séquence d'acides aminés succinyl-AAVALLPAVLLALLAPKNNLKECGLY.

7. Agent thérapeutique selon les revendications 1 à 6, comprenant en outre un diluant ou un vecteur pharmaceutiquement acceptable.

8. Agent thérapeutique selon les revendications 1 à 7, dans lequel ledit agent thérapeutique se trouve sous une forme posologique adaptée pour l'administration topique sur l'oeil, sur la peau ou sur la membrane muqueuse d'un sujet, ou sous une forme posologique adaptée pour l'administration par inhalation ou pour l'administration intranasale, ou sous une forme posologique adaptée pour une administration systémique orale ou parentérale.
